(19) Europäisches Patentamt
European Patent Office
Office européen des brevets

(11) EP 3 445 371 B1

(12) FASCICULE DE BREVET EUROPEEN

(45) Date de publication et mention de la délivrance du brevet:
**03.06.2020 Bulletin 2020/23**

(21) Numéro de dépôt: **17719527.8**

(22) Date de dépôt: **18.04.2017**

(51) Int Cl.:
***A61K 31/568*** *(2006.01)* ***A61K 31/5685*** *(2006.01)*
***A61K 31/57*** *(2006.01)* ***A61K 31/565*** *(2006.01)*
***A61K 31/567*** *(2006.01)* ***A61P 9/00*** *(2006.01)*
***A61P 9/06*** *(2006.01)* ***A61P 43/00*** *(2006.01)*

(86) Numéro de dépôt international:
**PCT/EP2017/059097**

(87) Numéro de publication internationale:
**WO 2017/182421 (26.10.2017 Gazette 2017/43)**

(54) **HORMONES STEROIDIENNES POUR LE TRAITEMENT ET LA PREVENTION DES TORSADES DE POINTES**

STEROIDALE HORMONE ZUR BEHANDLUNG UND PRÄVENTION VON WELLENBURST-ARRHYTHMIEN

STEROIDAL HORMONES FOR THE TREATMENT AND PREVENTION OF WAVE BURST ARRHYTHMIA

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **19.04.2016 FR 1653468**

(43) Date de publication de la demande:
**27.02.2019 Bulletin 2019/09**

(73) Titulaires:
- **Assistance Publique-Hôpitaux de Paris**
  **75004 Paris (FR)**
- **Institut National de la Santé et de la Recherche Médicale (INSERM)**
  **75013 Paris (FR)**
- **Sorbonne Université**
  **75006 Paris (FR)**

(72) Inventeurs:
- **SALEM, Joe-Elie**
  **75015 PARIS (FR)**
- **FUNCK-BRENTANO, Christian**
  **75014 PARIS (FR)**
- **BACHELOT, Anne**
  **75475 PARIS Cedex 10 (FR)**
- **WAINTRAUB, Xavier**
  **75475 PARIS Cedex 10 (FR)**

(74) Mandataire: **Flesselles, Bruno F.G.**
**BF IP**
**36 rue Jean de la Fontaine**
**75016 Paris (FR)**

(56) Documents cités:
- **SEDLAK ET AL: "ORAL CONTRACEPTIVE USE AND THE ECG: EVIDENCE OF AN ADVERSE QT EFFECT ON CORRECTED QT INTERVAL", ANNALS OF NONINVASIVE ELECTROCARDIOLOGY,, vol. 18, no. 4, 1 juillet 2013 (2013-07-01), pages 389-398, XP002764381, cité dans la demande**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).



Printed by Jouve, 75001 PARIS (FR)

## Description

## DOMAINE TECHNIQUE

La présente invention se rapporte au domaine de la cardiologie, et plus particulièrement à un nouveau procédé permettant de prévenir ou diminuer l'importance des troubles de la repolarisation et donc le risque de torsades de pointes après la survenue d'un facteur favorisant ou déclenchant endogène ou exogène comme la prise d'un médicament.

## ETAT DE LA TECHNIQUE ANTERIEUR

On appelle torsades de pointes un type particulier de tachycardie ventriculaire, un trouble excessif du rythme des ventricules cardiaques se traduisant par des accélérations du rythme cardiaque à type de tachycardie (différent néanmoins de la fibrillation ventriculaire).

Il existe deux types de torsades de pointes : les torsades à QT long et les tachycardies ventriculaires polymorphes à QT intercritique normal avec aspect de torsade.

Le rôle des hormones sexuelles dans la modulation du QT ou du QTc, QT corrigé par rapport à la fréquence cardiaque, selon les méthodes connues dans l'art, telles les méthodes de Fridericia ou de Bazett) a été étudié en profondeur, avec des résultats qui ne sont pas toujours similaires selon les études. D'une façon générale, toutefois, l'œstradiol est considéré comme prolongeant le QTc, tandis que la testostérone ou la progestérone diminuent le QTc. D'autres facteurs endogènes comme l'hypokaliémie, l'hypocalcémie, l'hypomagnésemie, les anomalies congénitales des canaux cardiaques, la bradycardie et l'âge déterminent aussi un allongement de la durée du QTc.

De nouveaux résultats rapportés notamment dans cette demande semblent montrer toutefois que la régulation est plus complexe, et met en jeu d'autres hormones telles que les gonadotrophines (notamment la FSH, hormone folliculostimulante) ou stéroïdiennes sexuelles ou les ratios des hormones stéroïdiennes sexuelles, comme le ratio progestérone/œstradiol chez les femmes et la présence de testostérone chez l'homme. Par ailleurs, d'autres hormones stéroïdiennes, structurellement similaires (telles que l'aldostérone qui prolonge le QTc), peuvent aussi moduler la repolarisation ventriculaire.

Il a été montré que les femmes en âge de procréer ont un intervalle QTc plus long durant la phase folliculaire dont leurs menstruations comparativement à la phase lutéale. Cet allongement de QTc est attribué actuellement à un défaut de production de progestérone durant ces périodes. Rodriguez et al (JAMA. 2001 Mar 14;285(10):1322-6) ont démontré que la variation de QTc après administration d'un médicament allongeant le QT chez les femmes en âge de procréer était corrélée négativement à la concentration plasmatique de progestérone et du ratio progestérone/estradiol. Odening et al (Heart Rhythm, Vol 9, No 5, May 2012) ont montré que l'œstradiol promeut une mort subite chez des lapins transgéniques présentant un QT long, tandis que la progestérone a un effet protecteur. Seth et al (JACC Vol. 49, No. 10, 2007:1092-8) ont rapporté que les femmes présentant un syndrome du QT long ont un risque cardiaque réduit pendant la grossesse, mais qui augmente en *post-partum.* De fait, durant la grossesse, le profil hormonal d'expression est profondément modifié avec en fin de grossesse une hyper-expression de progestérone aboutissant à des concentrations exprimées jusqu'à 1000 fois supérieure à celles retrouvées avant la grossesse. Par ailleurs, durant la grossesse, la production de FSH s'effondre et les concentrations deviennent quasi-indétectables. En *post-partum,* la progestérone chute rapidement et l'inhibition de la FSH est levée progressivement avec un retour à des concentrations pré-grossesse dès le premier mois suivant l'accouchement. Les inventeurs supposent que ces différentes variations hormonales pourraient expliquer les observations rapportées avec une augmentation du QTc en post-partum par rapport au 3$^{ème}$ trimestre de grossesse.

Il existe un grand nombre de médicaments induisant une augmentation du QTc, et qui sont donc à risque de mener à des torsades de pointes chez les patients, d'autant plus qu'ils ont un risque endogène prédisposant. Dans la grande majorité des cas, le mécanisme d'action de cet effet indésirable est l'inhibition d'un canal potassique impliqué dans la repolarisation ventriculaire appelé IKr qui induit une prolongation de la phase de repolarisation et se traduit électrocardiographiquement notamment par un allongement de l'intervalle QT corrigé sur la fréquence cardiaque (QTc).

Ainsi que mentionné, dans Sekarski et al (Paediatrica, Vol. 19 No. 4 2008), l'allongement du QT constitue ainsi, actuellement, la cause la plus fréquente de restriction d'usage et de retrait de médicaments du marché. Parmi les médicaments ainsi retirés du marché, cette publication cite notamment le cisapride, la terfénadine, le dropéridol ou le sertindole.

Ces phénomènes sont particulièrement observés avec les antiarythmiques de classe III, qui bloquent en priorité les canaux potassiques, allongeant ainsi la repolarisation. Les antiarythmiques de classe III actuellement sur le marché possèdent tous une structure de base incluant un groupement méthanesulfoaniline (ou un bio-isostère de celui-ci). On peut ainsi citer l'amiodarone (Cordarone®), l'azimilide, le brétylium, le clofilium, le dofétilide, l'ibutilide (Corvert®), le sématilide le sotalol (Sotalex®) et le dronédarone (Multaq®).

La publication ci-dessus présente, dans les tableaux 1 et 2, un certain nombre de médicaments connus pour

allonger le QT. Cette publication rappelle également que le « University of Arizona Center for Education and Research on Therapeutics » a établi et maintient une base de données de ces médicaments que l'on peut retrouver sur leur site internet www.torsades.org ou https://crediblemeds.org/.

Le site internet, http://www.urgences-serveur.fr/prise-en-charge-des-torsades-de,1543.html cite, quant à lui, un certain nombre de médicaments associés au développement de torsades de pointes.

Ceux ayant une action directe sur les canaux potassiques :

Antiarytmiques :

Classe Ia : quinidine, disopyramide
Classe III : sotalol, ibutilide, amiodarone, dofetilide

Antibiotiques : Macrolides : Erythromycine, Clarithromycine
Antifongiques : Ketoconazole, Itraconazole, Fluconazole
Psychotropes : Halopéridol, Phénothiazines, Lithium, Methadone
Antidépresseurs tricycliques : Imipramine, Amitriptyline
Antihistaminiques : Astemizole, Terfenadine
Gastrocinétiques : Cisapride
Hypolipémiants : probucol

Ceux interférant avec le métabolisme hépatique

Substrats terfenadine, astemizole, carbamazepine, ciclosporine, cisapride, sertraline
Inhibiteurs : erythromycine, clarithromycine, fluconazole, itraconazole

L'accès de torsade de pointes (tachycardie ventriculaire polymorphe) dure généralement quelques secondes et se résout spontanément. Toutefois, du fait que le cœur, battant à une fréquence supérieure à 200 pulsations par minutes n'a plus le temps d'assurer sa fonction hémodynamique, les torsades de pointes équivalent à un arrêt cardiaque. Le patient peut donc faire un malaise brutal qui peut être de courte durée, avec sensation de perte de connaissance, voire perdre brièvement connaissance (type syncope) tant que dure le trouble. Le patient présentera une pâleur pendant cet accès. Dans des formes extrêmes, l'accès de torsade de pointes ne se réduira pas spontanément et pourra évoluer vers une fibrillation ventriculaire et de manière ultime vers la mort.

La prise en charge d'un patient ayant eu un accès de torsade de pointes comporte essentiellement une recharge en potassium (KCl 3-6 g/j) et en magnésium (sulfate de magnésium IV : 3g IVD puis 6-12 g/24H).

On peut aussi mettre en place un entraînement électro-systolique si nécessaire particulièrement en cas de bardycardie et un transfert en unités de soins intensifs de cardiologie est la règle.

Il convient également d'arrêter la prise des médicaments pouvant allonger le QT (voir ci-dessus).

À plus long terme, on cherchera à identifier et corriger les facteurs de prédisposition (bradycardie, hypokaliémie, hypomagnésémie, cardiopathie congénitale sous-jacente).

Il existe un besoin d'améliorer la prise en charge des patients ayant présenté un accès de torsade de pointes, particulièrement dans les formes aigues récidivantes ou subintrantes appelées « orage rythmique » ainsi que de mettre en place une prévention de l'occurrence de ces événements à plus long terme. On définit un orage rythmique comme la survenue d'au moins 2 épisodes en moins de 24 heures ou 3 épisodes en moins de 48 heures de troubles du rythme ventriculaire nécessitant un traitement par le défibrillateur.

Selon Halimi (Médecine des maladies Métaboliques - Mars 2013 - Vol. 7 - N°), « le syndrome de mort subite dans son lit, « Dead-in-bed » *syndrome, concerne de rares sujets diabétiques de type 1, très jeunes et sans signe avant-coureur, sinon une fréquence élevée d'hypoglycémies sévères. Ces morts inexpliquées, qui sont jusqu'à 10 fois plus fréquentes que dans la population appariée non diabétique, seraient dues à l'enchaînement hyperinsulinisme, hypoglycémie, hypokaliémie, parallèlement à l'activation du système sympathique, et à un terrain prédisposant à déclencher des troubles du rythme et/ou de conduction graves. Mais l'hypoglycémie nocturne sévère est fréquente, et n'entraîne qu'exceptionnellement de telles conséquences dramatiques. L'hypoglycémie entraîne un allongement de l'intervalle QT, systématiquement, mais dont l'ampleur varie selon les individus, probablement du fait de prédispositions génétiques, comme dans les syndromes QT long familiaux. »* Il apparaît donc qu'un traitement préventif pouvant réduire la durée du QT serait potentiellement positif pour de tels patients.

Sedlak et al (Ann Noninvasive Electrocardiol. 2013 Jul;18(4):389-98) rapportent les résultats d'une étude transversale épidémiologique afin d'évaluer la relation entre les contraceptifs oraux et l'intervalle QTc. Cette étude est de bas niveau de preuve, notamment du fait que les groupes exposés aux différents types de pilules sont très différents entre eux, et présentent de nombreuses comorbidités. Par ailleurs, les mesures du Qtc sont automatiques sans vérification manuelle, et effectuées sur des tracés réalisés dans le cadre du soin courant et non dans l'objectif d'une recherche déterminée *a priori.* Il existe de très nombreux biais non pris en compte dans cette étude du fait de son caractère rétrospectif, en particulier comme l'absence d'information détaillée et de prise en compte des co-traitements pris par les sujets au moment de leur mesure électrocardiographique.

Ainsi, l'amplitude des effets différentiels mis en évidence dans cette étude est minime (4ms), et à la limite de la pertinence clinique (de fait, pour l'analyse des médicaments, on considère que ceux-ci n'ont pas de risque d'induire une repolarisation cardiaque tant que l'allongement du Qtc est inférieur à 5 msec) et sur une mesure du Qtc basal (c'est-à-dire sans stimulation avec un composé augmentant le Qtc) malgré des effectifs très importants (env 400 000 ECGs). Par ailleurs, Sedlak et al n'ont pas de groupe de patients prenant des pilules avec le gestodène, en tant que comparatif.

Il convient également de noter qu'il n'a pas été mis en évidence de différences entre les longueurs de QTc en fonction type de pilules en cas de co-traitement (Table 5 et discussion).

Ces résultats diffèrent donc de ceux rapportés dans la présente demande qui sont basés sur une étude avec absence de différences démographiques en fonction du type de pilule, et la soumission des patients à un stimulus pharmacologique standardisé connu pour favoriser les torsades de pointes. La présente demande montre que des différences de plus de 5 ms (environ 7ms) sont observées en fonction du type de pilules ajouté aux 20 ms moyen observé avec le sotalol, malgré des effectifs faibles par groupe (n=50).

Par ailleurs, il est rappelé que le QTc est associé au risque de torsades de pointes, mais n'est pas spécifique des TDP induites par les médicaments. La présente demande montre également que le type de pilules influence une série d'autres marqueurs associés, plus spécifiques d'une inhibition d'IKR (mécanisme d'action supposé des torsades de pointes induites par les médicaments). Il s'agit notamment de différence concernant l'apparition de notch et la modification de PC1 (nouveau marqueur composite plus pertinent de l'inhibition d'IKr).

Enfin, Sedlak *et al* ne parlent aucunement du potentiel thérapeutique des pilules dans la prévention ou le traitement des torsades de pointes.

Meriggiola et al (J Clin Endocrinol Metab. 2005 Apr;90(4):2005-14) décrivent l'utilisation de l'énanthate de noréthistérone et de testostérone. L'énanthate de noréthistérone n'est pas inclus dans la formule générale (I), ci-dessous.

**EXPOSE DE L'INVENTION**

L'invention se rapporte à un composé de formule (I) pour son utilisation pour le traitement et la prévention de la récidive des torsades de pointes.

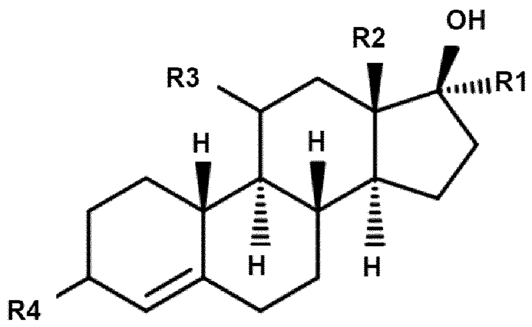

(I)

Dans laquelle

- R1 est choisi parmi H, $CH_3$, $CH_2$-$CH_3$, CH=$CH_2$, C≡CH
- R2 est choisi parmi $CH_3$, $CH_2$-$CH_3$, CH=$CH_2$, C≡CH
- -R3 est choisi parmi -H, -$CH_3$, =$CH_2$ (la liaison entre le carbone du cycle et R3 étant alors une liaison double), ≡CH (la liaison entre le carbone du cycle et R3 étant alors une liaison triple)
- -R4 est choisi parmi -H, -OH et =O (la liaison entre le carbone du cycle et R4 étant alors une liaison double)

Dans un mode de réalisation préféré,

- R1 est C≡CH
- R2 est choisi parmi $CH_3$, $CH_2$-$CH_3$
- -R3 est choisi parmi -H, =$CH_2$ (la liaison entre le carbone du cycle et R3 étant une liaison double)
- -R4 est choisi parmi -H et =O (la liaison entre le carbone du cycle et R4 étant une liaison double)

Dans un mode de réalisation préféré, le composé de formule (I) est le lévonorgestrel de formule (II) :

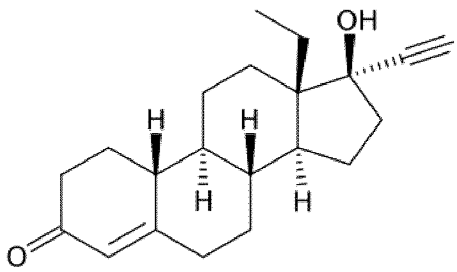

(II)

Dans ce mode de réalisation, R1 est C≡CH, R2 est $CH_2$-$CH_3$, -R3 est -H et -R4 est =O.
Il convient de noter que le lévonorgestrel peut être utilisé pur ou substantiellement pur, ou dans un mélange racémique (norgestrel) avec le composé isomère non actif.
Dans un autre mode de réalisation, le composé de formule (I) est la noréthistérone de formule (III) :

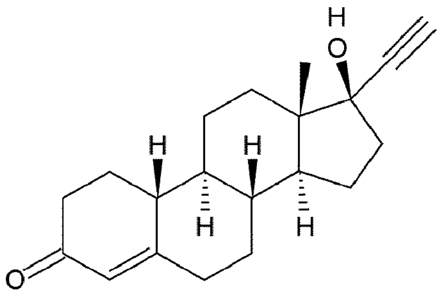

(III)

Dans ce mode de réalisation, R1 est C≡CH, R2 est $CH_3$, -R3 est -H et -R4 est =O.
Dans un autre mode de réalisation, le composé de formule (I) est le désogestrel de formule (IV)

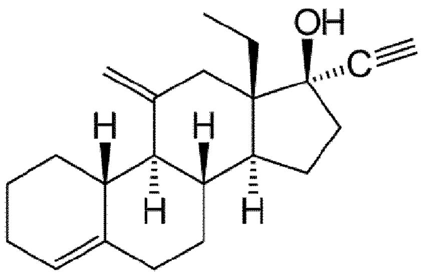

(IV)

Dans ce mode de réalisation, R1 est C≡CH, R2 est $CH_2$-$CH_3$, -R3 est =$CH_2$ et -R4 est -H
Dans un autre mode de réalisation, le composé de formule (I) est l'étonoestrel de formule (V) (métabolite actif du désogestrel)

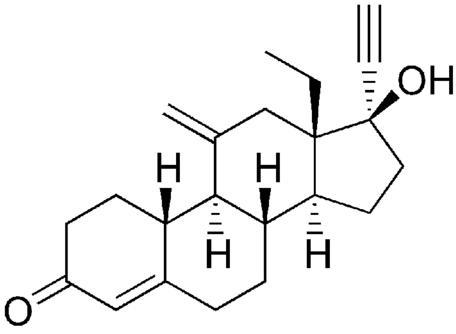

(V)

Dans ce mode de réalisation, R1 est C≡CH, R2 est $CH_2$-$CH_3$, -R3 est =$CH_2$ et -R4 est =O.

Dans la mise en œuvre de la présente invention, on utilise préférentiellement des progestatifs présentant un climat progestatif et présentant également très préférentiellement une activité androgénique. Les progestatifs utilisés dans le cadre de la présente invention sont donc préférentiellement dérivés de la 19 nortestostérone (nandrolone) de formule (VI), qui pourrait éventuellement être utilisée dans le cadre de l'invention.

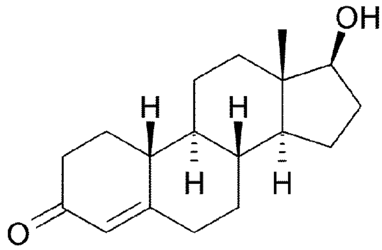

(VI)

Dans ce mode de réalisation, R1 est H, R2 est $CH_3$, -R3 est -H et -R4 est =O.

On pourrait toutefois aussi utiliser la médroxyprogestérone (17a-hydroxy-6a-méthylprogestérone), et en particulier l'acétate de médroxyprogestérone de formule (VII) dans le cadre de la présente invention.

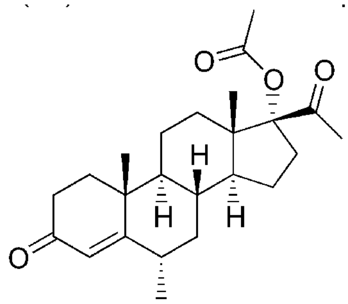

(VII)

En revanche, on évitera d'utiliser le diénogest ou le norgestimate qui présentent une faible activité androgénique, voire une activité anti-androgénique.

Il est rappelé qu'un progestatif est une hormone stéroïdienne d'action similaire à la progestérone, qui est présente les effets suivants en expérimentation chez un animal de laboratoire :

- effet lutéomimétique (test de Clauberg) : différenciation sécrétoire sur une muqueuse utérine correctement préparée par les œstrogènes
- effet progestogène : maintien de la gestation malgré ablation du corps jaune de l'ovaire.

Ces propriétés sont bien connues de l'homme du métier et peuvent donc aisément être testées (voir https://fr.wikipedia.org/wiki/Progestatif)

L'activité androgénique peut aisément être évaluée par des méthodes connues dans l'art, en étudiant la capacité du progestatif à se fixer à, et à activer le récepteur des androgènes (NR3C4).

Il est préféré que le composé utilisé présente une activité antigonadotrope la plus forte possible sur l'axe hypothalamo-hypophysaire. Ainsi, il existe une association positive entre Qtc et FSH (Abehsira et al, J Clin Endocrinol

Metab. 2016 Jul;101(7):2776-84). Un des mécanismes d'action des pilules est l'activité antigonadotrope (baisse centrale de FSH et LH) mais les pilules ne sont pas toutes équivalents sur ce point. Il est préféré, pour la prévention d'une torsade de pointes, que le composé utilisé présente une activité intrinsèque progestative et androgénique forte associée à une activité importante antigonadotrope (menant à une baisse de la FSH).

Le composé de formule (I) est donc utilisé pour le traitement et la prévention de la récidive des torsades de pointes.

Par traitement et prévention de la récidive d'une torsade de pointes, on entend désigner le traitement effectué après une crise de torsade de pointe ou pendant un orage rythmique. Ainsi, administrera le composé de formule (I) à un patient dans les trois jours suivant la survenue d'un épisode de torsade de pointes chez ce patient, de préférence, dans les 48 heures ou les 24 heures suivant cet épisode. On préfère toutefois utiliser le composé de formule (I) le plus rapidement possible après l'épisode de torsade de pointes, au même titre que les autres traitements décrits plus haut.

Dans ce mode de réalisation, le composé de formule (I) est utilisé pour traiter la phase aigüe suivant la torsade de pointes, ou durant un épisode d'orage rythmique. On traitera donc le patient après que celui-ci a présenté un épisode de torsade de pointes, pendant une durée inférieure ou égale à un mois, généralement pendant une durée inférieure ou égale à 15 jours.

Le composé de formule (I) aidera à réduire l'inhibition du canal potassique IKr et à rétablir la repolarisation ventriculaire.

Dans ce mode de réalisation, on utilisera le composé de formule (I) à une dose comprise entre 30 μg/jour et 3 mg/jour. Cette dose pourra être administrée en une ou plusieurs fois.

Dans un autre mode de réalisation, l'invention se rapporte à un composé de formule (I), tel que défini ci-dessus, pour la prévention primaire et secondaire de survenue d'épisodes de torsades de pointes chez un patient.

On pourra aussi utiliser la médroxyprogestérone (17a-hydroxy-6a-méthylprogestérone), et en particulier l'acétate de médroxyprogestérone de formule (VII) dans le cadre de la présente invention pour cet aspect de l'invention.

Par prévention primaire, on entend désigner le fait que le patient n'a pas présenté d'événement de torsade de pointes préalablement au début du traitement.

Par prévention secondaire, on entend désigner le fait que le patient a déjà présenté au moins un événement de torsade de pointes, mais qu'il n'est plus en orage rythmique ou que cet épisode a été présenté au moins un mois avant l'initiation du traitement.

Dans ce mode de réalisation, le patient peut présenter une prédisposition à la survenue de torsades de pointes. Ceci est notamment le cas lorsque le patient présente au moins un des symptômes suivants :

- un syndrome du QT long
- une hypokaliémie
- une hypomagnésie
- une bradycardie
- Une arythmie cardiaque paroxystique
- une cardiopathie sous-jacente notamment ischémique
- une hypogonadie chez l'homme
- un traitement par un médicament augmentant le QT
- un déficit constitutif en progestérone chez la femme
- un diabète, préférentiellement de type I

Ainsi, l'invention se rapporte à un composé de formule générale (I) pour son utilisation pour la prévention de la mort subite chez un patient diabétique.

L'invention se rapporte également à une composition contenant un composé de formule générale (I) et l'IGF-I (*insulin-like growth factor*-1) pour son utilisation simultanée, séparée ou étalée dans le temps pour la prévention de la mort subite chez un patient diabétique. L'IGF-I est un peptide linéaire de 70 acides aminés constitué de 4 domaines (B, C, A, D) dont les domaines B et A sont similaires à ceux de l'insuline, découverte et caractérisée en 1987.

L'invention se rapporte également à une composition contenant un composé de formule générale (I) et un androgène son utilisation simultanée, séparée ou étalée dans le temps pour la prévention de la mort subite chez un patient diabétique, le patient étant de sexe masculin.

On peut donc envisager, dans le cadre de l'invention, une composition contenant à la fois un composé de formule générale (I) et un androgène, utilisable notamment par voie orale. L'androgène est notamment tel que décrit plus bas.

Dans ce mode de réalisation, la quantité quotidienne de composé de formule (I) administrée au patient est comprise entre 30 μg et 300 μg, pour un traitement au long cours (c'est-à-dire un traitement pendant au moins 2 mois). Il convient de noter que l'on peut ne pas administrer de composé de formule (I) au patient pendant quelques jours par période de 28 jours. La quantité peut toutefois être très supérieure (500 μg à 1 mg par jour) pour certains composés de

formule (I), en particulier pour la noréthistérone.

Il convient de noter que les composés cités ci-dessus sont actuellement utilisés en tant que progestatifs dans le cadre d'une contraception orale chez les femmes.

Afin de maintenir les règles chez ces patientes, celles-ci ne prennent pas de contraceptif pendant quatre à cinq jours tous les 28 jours.

Dans le cadre de la méthode de prévention de torsades de pointes, il est toutefois préférable de donner les composés mentionnés ci-dessus en continu, c'est-à-dire sans arrêt de la thérapie au cours du temps.

On peut toutefois envisager, si le besoin de la patiente le requiert, d'interrompre la thérapie quelques jours (4 ou 5 jours) par mois, tous les 28 jours. Ceci maintiendra les règles chez les patientes.

Un autre mode de réalisation de l'invention se rapporte donc à un composé de formule générale (I), tel que défini ci-dessus, ainsi que tout autre composé décrit ci-dessus, pour son utilisation dans un schéma de contraception orale chez une femme, ladite femme ayant un risque de présenter un épisode de torsade de pointes.

Pour de telles patientes, il est en effet préférable de choisir une composition de contraception orale, contenant un progestatif tel que mentionné ci-dessus, plutôt qu'une composition à climat estrogénique (en particulier contenant du gestodène, du norgestimate ou de la drospirénone).

Il est toutefois entendu que la prescription de contraception doit prendre en compte l'ensemble des caractéristiques de la patiente et que le praticien adaptera cette prescription au cas par cas.

Dans un mode de réalisation préféré, le composé de formule générale (I) se présente sous une forme adapté à une administration par voie orale. De telles compositions existent déjà dans l'art (pilules contraceptives), et la prise quotidienne d'une seule pilule adéquatement dosée permet de délivrer la dose nécessaire, en particulier dans le cas d'un traitement au long cours pour prévenir la survenue d'événements de torsades de pointes. Les pilules existant sur le marché contiennent généralement un estrogène, en plus du composé de formule générale (I) ou du progestatif décrit ci-dessus. Il est parfois préférable de supprimer cet estrogène dans le cadre des méthodes et traitements décrits dans la présente demande, afin d'augmenter l'effet protecteur du progestatif et composé de formule (I).

Ainsi, dans ce mode de réalisation, la composition administrée au patient ne contient, en tant que principe actif, que le composé de formule générale (I). Ceci signifie que les autres éléments contenus dans la composition sont des excipients qui n'ont pas d'effet connu ou répertorié sur les récepteurs à la progestérone, aux androgènes ou à l'estradiol, ou d'autre effet connu ou répertorié dans le domaine cardiovasculaire. D'une façon générale, ces excipients n'ont pas effet physiologique connu ou répertorié. En particulier, la composition ne contient aucune autre hormone naturelle ou synthétique présentant un effet connu ou répertorié sur les récepteurs à la progestérone, aux androgènes ou à l'estradiol.

Dans un autre mode de réalisation, la composition peut contenir un estrogène (tel que l'éthinylestradiol, estradiol, ou le valérate d'estradiol). Dans ce mode de réalisation, les doses unitaires des compositions contiennent généralement de l'ordre de 15-40 $\mu$g d'estrogène, alors qu'elles contiennent entre 50 et 200 $\mu$g de composé de formule (I). On peut même envisager des pilules contenant 500 $\mu$g à 1mg de noréthistérone avec environ 35 $\mu$g d'estrogène (éthinylestradiol).

Dans le cadre des traitements décrits dans la présente invention, on pourra aussi utiliser des compositions dans lesquelles les doses d'estrogène sont inférieures à celles mentionnées ci-dessus.

Dans le cas d'un traitement d'un événement, en cas notamment d'orage rythmique, on peut donner des pilules déjà existantes, telles que le Norlevo®, dont chaque dose unitaire contient 1,5 mg de lévonorgestrel, dont on peut prendre deux pilules en une ou deux prises.

D'une façon générale, la composition selon l'invention peut se présenter sous toute autre forme connue dans l'art. En particulier, elle peut se présenter sous la forme de gélules, de comprimés (pelliculés ou non), de pilules ou de tablettes. Dans un autre mode de réalisation, elle se présente sous la forme d'une composition liquide, telle qu'un sirop.

Dans le cas où la composition se présente sous une forme solide, on peut utiliser tout excipient connu dans l'art, tel que le talc (E553b), la cellulose microcristalline, le lactose, l'amidon (en particulier de maïs), le stéarate de magnésium (E572), l'acide stéarique (E570).la cellulose microcristalline. Lorsque la composition se présente sous la forme d'un comprimé pelliculé, ladite pellicule peut être formée de toute substance connue dans l'art, telle que l'hypromellose (E464), l'éthylcellulose, la macrogol, le Talc (E553b), le dioxyde de titane (E171), l'oxyde de fer (E172).

Les compositions pourraient éventuellement être à libération retardée (slow release), même si de telles compositions ne sont pas préférées, du fait du besoin de délivrance rapide du principe actif en cas d'orage rythmique, et du second effet (contraceptif) en cas de traitement au long terme.

Dans un autre mode de réalisation, le composé de formule (I) se présente sous une forme adapté à une administration par voie injectable (en particulier intraveineuse). Ce cas est particulièrement adapté lors du traitement d'épisode de torsades de pointes, en cas d'un événement récent ou d'orage rythmique.

Dans d'autres modes de réalisation, on peut délivrer les composés par implant sous-cutané. De tels implants sont utilisés en contraception, et contiennent les composés mentionnés ci-dessus. Une fois l'implant mis en place, le composé se diffuse directement dans le sang. Les implants aujourd'hui sur le marché (Nexplanon®) contiennent généralement suffisamment d'hormone pour être efficace pendant trois ans.

On peut aussi délivrer les composés mentionnés ci-dessus *via* des stérilets imprégnés, en particulier le stérilet

MIRENA®, inséré dans la cavité utérine où il délivre le lévonorgestrel pendant 5 ans.

On peut aussi délivrer les composés via un anneau vaginal imprégné, qui est à remplacer toutes les trois semaines.

Les composés décrits ci-dessus peuvent également être délivrés via des compositions topiques (pommades ou crèmes, notamment vaginales).

On peut aussi envisager que ces composés soient délivrés par l'intermédiaire d'un patch transdermal.

D'une façon générale, il est très préféré que le patient soit de sexe féminin. Toutefois, le composé de formule (I) peut aussi être utilisé chez l'homme, notamment et de préférence lorsqu'on administre un androgène en combinaison avec de composé de formule (I).

L'invention se rapporte ainsi à une composition contenant un composé de formule (I) et un androgène (un composé hormonal stéroïdien activant le récepteur NR3C4) pour son utilisation simultanée, séparée ou étalée dans le temps pour le traitement ou la prévention de torsades de pointes.

Dans un mode de réalisation préféré, ledit androgène est choisi dans le groupe constitué de la testostérone, la déhydroépiandrostérone, la Δ4-androstènedione, l'androstérone et la dihydrotestostérone.

Une telle thérapie de combinaison peut être utilisée chez l'homme et la femme.

La présente description divulgue également l'utilisation d'un composé de formule générale (I) pour la préparation d'un médicament destiné au traitement des torsades de pointes.

La présente description divulgue également l'utilisation d'un composé de formule générale (I) pour la préparation d'un médicament destiné à la prévention (primaire ou secondaire) de survenue d'épisodes de torsades de pointes ou d'apparition de troubles de la repolarisation suggestifs d'un haut risque de torsades de pointes.

La présente description divulgue également à l'utilisation d'un composé de formule générale (I) pour la préparation d'un contraceptif oral destiné aux femmes présentant un risque de survenue de torsades de pointes ou d'apparition de troubles de la repolarisation suggestifs d'un haut risque de torsades de pointes.

La présente description divulgue également à l'utilisation d'un composé de formule générale (I) pour la préparation d'un médicament destiné à réduire le QT chez un patient.

La présente description divulgue également une méthode de traitement d'un patient ayant présenté un épisode d'une torsade de pointe, ou en orage rythmique, comprenant l'administration d'une quantité adéquate d'un composé de formule (I) au patient.

La présente description divulgue également une méthode de prévention (primaire ou secondaire) de survenue d'épisodes de torsades de pointes chez un patient ou d'apparition de troubles de la repolarisation suggestifs d'un haut risque de torsades de pointes, comprenant l'administration d'une quantité adéquate d'un composé de formule (I) au patient.

La présente description divulgue également une méthode pour réduire le QT chez un patient, comprenant l'administration d'une quantité adéquate d'un composé de formule (I) au patient.

Plus spécifiquement, la description divulgue l'administration, à un patient le nécessitant, d'un composé de Formule (I) ou plus spécifiquement décrit plus haut, en quantité suffisante pour traiter ou prévenir un ou plusieurs épisode(s) de torsades de pointes, et/ou diminuer le QTc chez ce patient. Ce composé peut être administré seul ou avec un autre composé (notamment tel que décrit plus haut, IGF-I ou à propriété androgénique), qui est utilisé simultanément, séquentiellement ou de manière espacée dans le temps. L'administration est effectuée ainsi que décrit plus haut, et en particulier par voie orale ou par voie sous-cutanée.

## BREVES DESCRIPTION DES FIGURES

Figure 1: caractéristiques des patients ayant reçu du sotalol, et de leur mode de contraception. H3 représente la valeur 3 heures après administration de sotalol. Statistiques: ns représente p>0,1. Différents tests (Chi2 test avec trend, One-way ANOVA avec post-test de Turkey ou Kruskal-Wallis avec post-test de Dunn) ont été utilisés. Les données quantitatives sont présentées en tant que moyenne ± écart type ou médiane avec écart interquartile.
*(pas d'hormones vs drospirénone), **(lévonorgestrel vs drospirénone), # (désogestrel vs gestodene), § (lévonorgestrel vs pas d'hormones), ‡(gestodene vs pas d'hormione).
La valeur PC1 est obtenue par l'analyse par composantes principales des variables ΔQTc (%), ΔTpTe (%) et ΔTAmp (%) et reflète quantitativement l'inhibition d'Ikr obtenue 3 heures après la prise de sotalol.

Figure 2 : Valeur PC1 trois heures après administration de 80mg de sotalol en fonction du type de pillules. Les statistiques (valeurs significatives) ont été obtenues par un test de Kruskal-Wallis avec post test de Dunn. Significativité avec seuil : p<0,05

Figure 3 : Valeur de la différence de QTc (ms) trois heures après administration de 80ms de sotalol en fonction du type de pillules. Les statistiques (valeurs significatives) ont été obtenus par ANOVA avec Turkey post test, p<0,01

Figure 4 : nombre et proportion de patientes avec un double pic de l'onde T induit par le sotalol en fonction du type de contraception

**EXEMPLES**

**Exemple 1 - Mise au point d'un marqueur représentatif de l'inhibition du canal potassique IKr**

Les résultats obtenus sur une cohorte de 995 volontaires en bonne santé, âgés de 18 à 60 d'origine européenne ou nord-africaine ont servi au développement d'un marqueur représentatif de l'inhibition du canal potassique IKr.

On a obtenus les résultats sur une cohorte d'exploration, ayant servi à identifier le marqueur décrit ci-après et une cohorte de réplication ayant servi à vérifier les résultats obtenus sur la cohorte d'exploration.

En bref, on a enregistré l'électrocardiogramme (ECG) des patients (chacun en triple, pendant 10 secondes) des patients après 10 minutes au repos sur le dos. On a ensuite donné une dose orale unique de 80 mg de sotalol aux patients et poursuivi le suivi ECG. Trois heures après la prise de sotalol, on a de nouveau enregistré l'ECG (triplicatas) après que les sujets sont restés couchés sur le dos pendant 10 minutes.

*Analyse des ECG*

L'augmentation du QT par le sotalol représente l'équivalent de la forme iatrogène congénitale du syndrome du QT long LQT2, on a vérifié l'apparition des différences classiques entre le tracé de base et le tracé trois heures après prise de sotalol sur l'ECG.

Les QTcF (QT corrigé par la formule de Frediricia), TpTe (durée entre le premier pic et la fin de l'onde T), TAmp (amplitude du premier pic de l'onde T) et la présence de doubles pics dans l'onde T (appelés *notch* en anglais) ont été quantifiés. La correction de Fridericia (QTcF) a été utilisée pour corriger le QT par rapport à la fréquence cardiaque, en accord avec les directives ICH E14. L'analyse des données d'ECG a été effectuée avec le logiciel CARDIABASE (Groupe Banook, Nancy, France).

TpTe a été mesurée par la méthode de tangente, en triplicata, sur un battement représentatif (représentation moyennée d'un ECG de 10 secondes) sur les dérivations V3, V4 et V5. En cas de double pic de l'onde T (notch), le pic pris en considération a été le premier pic, même si l'amplitude de l'onde T était moins importante pour ce pic. La valeur moyenne obtenue a été retenue. En cas d'impossibilité de mesure de TpTe sur V3, V4 ou V5, on a, à la place, effectué la mesure sur V2 (pour V3), ou V6 (pour V4/V5).

Afin de mesurer, TAmp, on s'est positionné sur l'endroit de l'amplitude maximale du premier pic de l'onde T sur un battement représentatif (représentation moyennée d'un ECG de 10 secondes) sur les dérivations DII, V2, et V3. On a calculé et retenu la moyenne des valeurs TAmp issues de ces trois dérivations, et mesurées sur trois différents ECG de 10 secondes (triplicatas). S'il n'était pas possible de mesurer une TAmp sur une de ces dérivations (notamment en raison d'une faible valeur (<0.1mV)), on a utilisé la dérivation V4.

QTcF a été mesurée par la méthode de la tangente sur la dérivation DII pour trois battements consécutifs et la valeur moyenne d'une évaluation en triplicata a été retenue.

On a pu vérifier que les mesures restaient cohérentes et reproductibles entre les évaluateurs.

Pour ces paramètres, on a mesuré la différence entre la valeur obtenue à H3 et la valeur de base. On a exprimé cette différence en pourcentage par rapport à la valeur de base, et on a effectué les calculs de telle sorte que la valeur calculée soit positive.

On a donc effectué les calculs suivants :

$$\Delta QTcF\ (\%) = (QTcF\ \text{à}\ H3 - QTcF\ basal) / (QTcF\ basal) \times 100$$

$$\Delta TpTe\ (\%) = (TpTe\ \text{à}\ H3 - TpTe\ basal) / (TpTe\ basal) \times 100$$

$$\Delta TAmp\ (\%) = (TAmp\ basal - TAmp\ \text{à}\ H3) / (TAmp\ basal) \times 100$$

Pour évaluer les doubles pics (notchs), tous les ECG ont été évalués indépendamment par deux investigateurs. Les patients ont été qualifiés de *notchers* ou *non-notchers,* en cas d'accord entre les deux investigateurs. En cas de discordance dans l'évaluation, les sujets n'ont pas été inclus dans ces groupes (seuls 10 sujets dans la cohorte d'évaluation et 8 sujets dans la cohorte de réplication n'ont pu être ainsi classifiés).

*Analyse en composantes principales*

Une analyse en composantes principales a été effectuée sur les valeurs calculées ΔQTcF, ΔTpTe et ΔTAmp, ce qui a permis de générer trois marqueurs alternatifs non corrélés PC1, PC2 et PC3, prenant mieux en compte l'information portée par les trois valeurs initiales corrélées. Les analyses statistiques ont été effectuées avec le logiciel R (https://www.r-project.org/). L'analyse en composantes principales (ACP ou PCA en anglais) est une méthode permettant de transformer des variables corrélées en nouvelles variables décorrélées les unes des autres

*Résultats*

Changements dans l'électrocardiogramme induits par le Sotalol

Les changements observés dans l'électrocardiogramme trois heures après administration du sotalol suggèrent une inhibition du canal IKr, avec des résultats et observations similaires dans les cohortes d'exploration et de réplication, quels que soient les paramètres qualitatifs ou quantitatifs évalués.

En accord avec des études précédentes, le sotalol induit également des changements absolus et relatifs dans la durée du QTcF (21.4±14ms et 5.5±3.5% respectivement).

Des changements typiques dans la forme de l'onde T ont également été observés, avec une augmentation du TpTe (14.2±15.6 vs 15.9±20.5%, p=ns (non significatif)) et une diminution de TAmp (13.6±15.7 vs 12.8±15.3%, p=ns) respectivement entre les cohortes d'exploration et de réplication.

On a toutefois observé une grande variabilité inter-sujets dans tous les changements observés. Les variations de TpTe, TAmp et QTcF entre H3 et le niveau basal présentent une autocorrélation dans la même proportion entre les deux cohortes.

Enfin, 40 (8 %) ou 51 (10 %) sujets présentaient un double pic (notch) dans chacune des deux cohortes. Il s'agissait presque exclusivement de femmes, et ces patients avaient un ΔTpTe, ΔQTcF et ΔTAmp supérieur à ceux observés pour les non-notchers (patients sans double pic).

Analyse en composantes principales

L'analyse en composantes principales a permis de générer trois nouvelles valeurs quantitatives, liées aux modifications de repolarisation ventriculaire induites par le sotalol.

Le premier composant (PC1) expliquant environ 65-67 % de la variance phénotypique reflète les modifications typiques après inhibition du canal IKr, c'est-à-dire l'augmentation de TpTe, QTcF et la diminution de TAmp (Tableau 1).

La variable PC1 considérée est la variable qui est la mieux corrélée à chacun des ΔQTc, ΔTamp et ΔTpTe. Cela signifie que le coefficient de corrélation que l'on peut calculer pour PC1 avec chacun des ΔQTc, ΔTamp et ΔTpTe est supérieur (en valeur absolue) au coefficient de corrélation que l'on calcule pour les autres composantes principales PC2 et PC3 pour le ΔQTc, ΔTamp ou ΔTpTe en question. Le tableau 1 des exemples montre bien que la valeur absolue du coefficient de corrélation de PC1 est supérieure à la valeur absolue du coefficient de corrélation des PC2 et PC3 calculé pour chacun des ΔQTc, ΔTamp et ΔTpTe. Selon la façon dont on effectue l'analyse en composantes principales, PC1 peut être corrélée positivement ou négativement aux ΔQTc, ΔTamp et ΔTpTe décrits ci-dessus.

La valeur PC1 est très bien associée à l'apparition de signe ECG déterminant une inhibition d'IKr, et présumés associés à un risque accru d'épisode de torsade de pointes à QT long.

Dans le cadre du calcul réalisé dans cette étude ayant permis de mettre au point le marqueur PC1, PC1 est corrélée de façon négative aux ΔQTc, ΔTamp et ΔTpTe. En conséquence, l'augmentation de ces valeurs mène à une diminution de PC1. On a observé une diminution significative de la valeur PC1 ($p<10^{-4}$) chez les patients présentant un double pic (*notchers*) par rapport aux patients ne présentant pas ce double pic (*non-notchers*) dans l'une ou l'autre des cohortes d'exploration (-2,5±1,5 vs 0,3±1,1) ou de réplication (-2,3±1,8 vs 0,3±1,1).

La valeur PC1 est donc une valeur alternative quantitative de l'inhibition de IKr.

Les autres composantes principales (PC2 et PC3) expliquaient respectivement environ 19-21% et 14% de la variance phénotypique.

Tableau 1. Corrélations (r) entre ΔTAmp, ΔTpTe, ΔQTcF et les composantes principales dans les cohortes d'exploration (n=489, partie supérieure du tableau) ou de réplication (n=495, partie inférieure du tableau)

| Corrélation(r) | ΔTAmp | ΔTpTe | ΔQTcF | PC1 | PC2 | PC3 |
|---|---|---|---|---|---|---|
| ΔTAmp | 1 | 0,59 | 0,40 | -0,83 | 0,36 | -0,43 |
| ΔTpTe | 0,58 | 1 | 0,45 | -0,85 | 0,23 | 0,47 |

(suite)

| Corrélation(r) | ΔTAmp | ΔTpTe | ΔQTcF | PC1 | PC2 | PC3 |
|---|---|---|---|---|---|---|
| ΔQTcF | 0,47 | 0,46 | 1 | -0,73 | -0,68 | -0,06 |
| PC1 | -0,84 | -0,84 | -0,78 | 1 | 0 | 0 |
| PC2 | 0,28 | 0,3 | -,063 | 0 | 1 | 0 |
| PC3 | -0,46 | 0,45 | 0,01 | 0 | 0 | 1 |

On observe bien que PC1 est corrélée dans le même sens avec ΔTAmp, ΔTpTe, et ΔQTcF (corrélée négativement dans cet exemple), ce qui n'est pas le cas pour PC2 ou PC3.

On observe également que la valeur absolue des coefficients de corrélation de PC1 avec ΔTAmp, ΔTpTe, et ΔQTcF est supérieure à celle des PC2 ou PC3 avec ces valeurs.

*Conclusion*

Comme attendu, une augmentation des TpTe, QTcF et une diminution de TAmp ont été observés entre le niveau basal et trois heures après prise de sotalol.

La nouvelle valeur PC1 obtenue par analyse en composantes principales de ΔQTcF, ΔTpTe et ΔTAmp explique l'essentiel de la variance totale des données « Δ » (ΔQTcF, ΔTpTe et ΔTAmp) et est ainsi associée au mécanisme commun d'augmentation de QTcF et TpTe, associé à la diminution de TAmpl.

En conséquence, PC1 est un marqueur quantitatif et intégratif de l'inhibition IKr induite par médicament.

**Exemple 2 - Le lévonorgestrel et le désogestrel ont une action plutôt protectrice sur l'inhibition du canal potassique IKr**

*Population*

L'étude a été réalisée sur 615 femmes en bonne santé, de type européen ou nord-africain, entre 18 et 60 ans, avec un QTcF < 450ms, et aucune maladie ou chronique connue, ou traitement chronique, excepté contraception, et sans antécédents familiaux de syndrome du QT long congénital, arythmie, ou mort subite.

*Mesure et calcul des différentes données QTcF, Tamp et TpTe*

Les QTcF, TAmp et TpTe ont été mesurés selon le même protocole que pour l'exemple 1, avant et trois heures après administration d'une dose orale unique de sotalol à 80 mg.

On a aussi calculé les ΔQTcF, ΔTpTe et ΔTAmp.

*Analyse en composantes principales*

Une analyse en composantes principales a été effectuée sur les ΔQTcF, ΔTpTe et ΔTAmp (logiciel XLStat, Addinsoft®) afin de générer trois nouvelles valeurs décorrélées PC1, PC2 et PC3.

*Résultats*

La variable PC1 ainsi obtenue (dont la valeur absolue des coefficients de corrélation avec ΔTAmp, ΔTpTe, et ΔQTcF est supérieure à celle des coefficients de corrélation pour PC2 ou PC3 avec ces valeurs) explique 63 % de la variance phénotypique observée (caractérisée par l'augmentation de QTcF et TpTe et la diminution de TAmp). Dans ce mode de réalisation, PC1 est corrélée positivement dans le même sens que ΔQTcF, ΔTpTe et ΔTAmp, c'est-à-dire qu'elle augmente lorsque ces valeurs augmentent (Tableau 2).

Tableau 2. Corrélation entre les variables et les facteurs obtenus après APC, et contribution à la variance totale de chacun de ces facteurs (n=498).

| | **PC1** | PC2 | PC3 |
|---|---|---|---|
| ΔQTcF (%) | **0.71** | 0.71 | -0.06 |
| ΔTpTe (%) | **0.85** | -0.24 | 0.48 |

(suite)

| | **PC1** | PC2 | PC3 |
|---|---|---|---|
| ΔTAmp (%) | **0.83** | -0.36 | -0.44 |
| Contribution à la variance totale | **63.2 %** | 22.7% | 14,1% |

La Figure 1 montre les différentes caractéristiques des patients ayant reçu le sotalol, ainsi que l'hormone contraceptive.

On observe:

- Les niveaux de base de QTcF, et les concentrations de sotalol dans le plasma trois heures après administration, ainsi que la kalémie n'étaient pas différents entre les sous-groupes fonction du type de contraception.
- L'augmentation de QTcF pour les patientes sous drospirénone était supérieure à celle observée pour les patientes sans contraception hormonale (de 6,6 ± 2,6msec) ou sous lévonorgestrel (de 7,0 ± 2,7msec) (Figure 3).
- Les patientes sous drospirénone et gestodène présentaient une incidence plus importante ($p<0.01$) de double pics 3 heures après administration de Sotalol (25,8% et 23,5% respectivement) que les patientes sous désogestrel (7,3%), lévonorgestrel (16,4%) ou sans contraception hormonale (13,4%) (Figure 4).
- La valeur de PC1 était significativement supérieure dans le groupe drospirénone que dans le groupe de patientes sans contraception orale (respectivement 0,56 [-0,13; 1,5] et 0,09[-0,58; 1,1], $p<0,05$) (Figure 2)
- La valeur de PC1 était plus modérée dans le groupe désogestrel et lévonorgestrel (respectivement 0,27[-0,47; 1,2] et 0,22 [-0,54; 1,2]) (Figure 2)

On voit donc que toutes les méthodes de contraception orale ne sont pas identiques en ce qui est des risques d'inhibition du canal IKr induit par la prise de médicaments.

La drospirénone présente le risque le plus important d'inhibition de IKr induit par médicaments, alors que le lévonorgestrel, et le désogestrel semblent être neutres, le lévonorgestrel étant plutôt protecteur pour ce qui est de l'augmentation du QT et le désogestrel étant plutôt protecteur pour ce qui est de l'apparition d'un double pic de l'onde T.

Les inventeurs font l'hypothèse que ceci est dû à l'effet à la fois progestatif et angrogénique de ces deux hormones, par rapport à la drospirénone, qui ne possède pas d'effet androgénique (tableau 3).

Si l'on diminue ou supprime les doses d'estrogènes (éthynil-estradiol) administrés en même temps que ces progestatifs, l'effet protecteur de ces progestatifs seraient encore plus marqué.

Tableau 3. Caractéristiques des différentes pilules orales pour contraception hormonale

| | Levonorgestrel | Desogestrel | Gestodene | Drospirenone |
|---|---|---|---|---|
| Nombre de patients | 137 | 41 | 51 | 62 |
| Utilisation concomitante EE (%) dose EE min/max* (μg/day) | 99 | 78 | 100 | 100 |
| | 20-35 | 20-30 | 15-35 | 20-30 |
| Dose PG min/max* (μg/day) | 30-175 | 75-150 | 60-75 | 3000 |
| Génération de pilules | 2nde | 3ème | | 4ème |
| Activité progestative | Haute | Haute | | Intermédiaire |
| Androgénicité | Haute | Intermédiaire / variable | | Anti-androgénique |
| Abréviations: EE: Ethynil-estradiol, PG: progestatif, *: en cas d'utilisation concomitante d'EE, excepté les jours sous placébo ou hors pilules | | | | |

## Exemple 3 - traitement d'une torsade de pointe par adminstration de testostérone

Un hypogonadisme périphérique a été identifié chez un homme atteint d'une cardiopathie ischémique et d'une maladie d'Erdheim-Chester en orage rythmique sur torsades de pointes avec QT long et notching (double pic de l'onde T) favorisées par la iatrogène et la bradycardie avec plus de 7 épisodes en moins d'une semaine.

De la testostérone (Androtardyl®, énantate de testostérone 250 mg/ml) a été administrée au patient à des quantités permettant de maintenir un niveau de testostérone permettant de corriger son hypogonadie, la bradycardie a été corrigée et un défibrillateur implantable a été posé en prévention secondaire.

Suite à l'introduction de la testostérone, la concentration circulante en testostérone biodisponible et sa repolarisation se sont normalisées.

Trois mois suivant l'introduction de testostérone, le contrôle du défibrillateur a confirmé l'absence de récidive de torsades de pointes malgré la réintroduction de médicament allongeant le QTc (Vémurafénob) dont le patient avait besoin pour sa maladie de fond (Erdheim-Chester).

Ce cas clinique est en faveur de l'efficacité d'une modulation hormonale pour le traitement et la prévention des Torsades de pointes..

**Conclusion**

L'exemple 3 est la première démonstration que la testostérone, au-delà de son effet discuté sur le QTc, est capable de traiter un patient présentant un orage rythmique de torsades de pointes.

L'exemple 2 montre que les pilules progestatives à effet androgénique peuvent être utilisées pour prévenir les épisodes de torsade de pointes, car contrôlant l'inhibition du canal IKr, ainsi que démontré par la faible augmentation de la valeur du marqueur PC1. Ainsi, cet exemple montre bien un effet protecteur de ces pilules, en présence d'une administration conjointe d'un médicament augmentant le risque de torsades de pointes.

Du fait des propriétés des pilules progestatives à effet androgénique montrées à l'exemple 2 et du résultat clinique de l'exemple 3, l'utilisation des pilules progestatives à effet androgénique peut être envisagée, en particulier chez les femmes, pour le traitement de torsades de pointes, lors de la prise en charge d'un patient venant de subir un épisode de torsade de pointes, ou en orage rythmique.

Il est toutefois préférable de diminuer la quantité en estrogène de ces pilules, et d'administrer des pilules à base de progestatifs purs, tels que décrits ci-dessus, afin d'amplifier l'effet dû à ces molécules.

**Revendications**

1. Composé de formule générale (I)

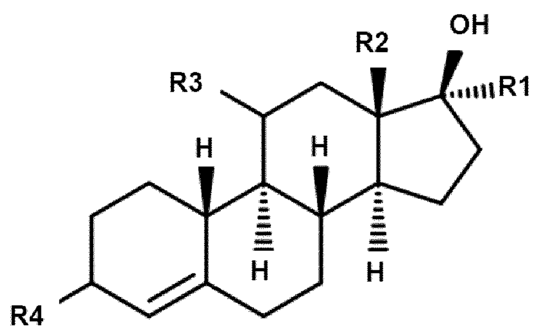

dans laquelle

- R1 est choisi parmi H, $CH_3$, $CH_2$-$CH_3$, CH=$CH_2$, C≡CH
- R2 est choisi parmi $CH_3$, $CH_2$-$CH_3$, CH=$CH_2$, C≡CH
- -R3 est choisi parmi -H, -$CH_3$, =$CH_2$ (la liaison entre le carbone du cycle et R3 étant alors une liaison double), ≡CH (la liaison entre le carbone du cycle et R3 étant alors une liaison triple)
- -R4 est choisi parmi -H, -OH et =O (la liaison entre le carbone du cycle et R4 étant alors une liaison double)

pour son utilisation pour le traitement des torsades de pointes.

2. Composé de formule générale (I) pour son utilisation pour la prévention de survenue d'épisodes de torsades de pointes.

3. Composé de formule générale (I) pour son utilisation pour la prévention de la mort subite chez un patient diabétique provoquée par la survenue de torsades de pointes.

4. Composé de formule générale (I) pour son utilisation selon l'une des revendications 1 à 3, **caractérisé en ce que** le patient présente une prédisposition à la survenue de torsades de pointes.

5. Composé de formule générale (I) pour son utilisation selon la revendication 4, **caractérisé en ce que** le patient suit un traitement par un médicament augmentant le QT.

6. Composé de formule générale (I) pour son utilisation selon l'une des revendications 1 à 5, **caractérisé en ce qu'**il est administré au patient après que celui-ci a présenté un épisode de torsade de pointes.

7. Composé de formule générale (I) pour son utilisation selon la revendication 1, **caractérisé en ce qu'**il est administré, pendant une durée inférieure ou égale à 15 jours, au patient après que celui-ci a présenté un épisode de torsade de pointes.

8. Composé de formule générale (I) pour son utilisation selon l'une des revendications 1, 4 à 6, dans leur dépendance à la revendication 1, **caractérisé en ce que** la quantité quotidienne de composé de formule (I) administrée au patient est comprise entre 30 µg et 3 mg.

9. Composé de formule générale (I) pour son utilisation selon la revendication 2 à 5, **caractérisé en ce que** la quantité quotidienne de composé de formule (I) administrée au patient est comprise entre 30 µg et 300 µg.

10. Composé de formule générale (I) pour son utilisation selon l'une des revendications 1 à 7, caractérisé en qu'il se présente sous une forme adaptée à une administration par voie orale.

11. Composé de formule générale (I) pour son utilisation selon l'une des revendications 1, 6 à 10, caractérisé en qu'il se présente sous une forme adaptée à une administration par voie injectable.

12. Composé de formule générale (I) pour son utilisation selon l'une des revendications 1 à 11, **caractérisé en ce que** le patient est de sexe féminin.

13. Composé pour son utilisation selon l'une des revendications 1 à 12, **caractérisé en ce qu'**il est choisi dans le groupe constitué du lévonorgestrel, de la noréthistérone, du désogestrel, de l'étonogestrel et de la 19 nortestostérone.

14. Composition contenant un composé de formule générale (I) et un androgène pour son utilisation simultanée, séparée ou étalée dans le temps pour le traitement ou la prévention de torsades de pointes.

15. Composition pour son utilisation selon la revendication 14, **caractérisée en ce que** ledit androgène est choisi dans le groupe constitué de la testostérone, la déhydroépiandrostérone, la Δ4-androstènedione, l'androstérone et la dihydrotestostérone.

16. Composition contenant un composé de formule générale (I) et l'IGF-I pour son utilisation simultanée, séparée ou étalée dans le temps pour la prévention de la mort subite chez un patient diabétique, provoquée par la survenue de torsades de pointes.

17. Composé de formule générale (I) et un androgène pour son utilisation pour la prévention de la mort subite chez un patient diabétique provoquée par la survenue de torsades de pointes, le patient étant de sexe masculin.

## Patentansprüche

1. Verbindung der allgemeinen Formel (I)

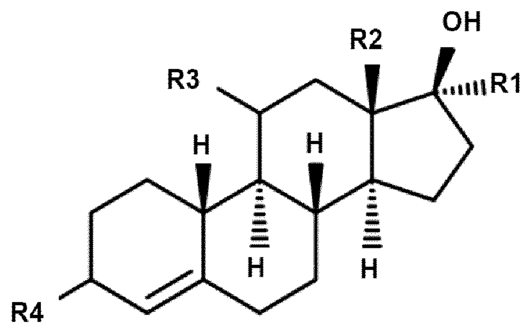

wobei

- R1 ist ausgewählt aus H, $CH_3$, $CH_2$-$CH_3$, $CH=CH_2$, $C\equiv CH$
- R2 ist ausgewählt aus $CH_3$, $CH_2$-$CH_3$, $CH=CH_2$, $C\equiv CH$
- R3 ausgewählt wird aus -H, -$CH_3$, =$CH_2$ (die Bindung zwischen dem Karbon des Ringes und R3 ist dann eine Doppelbindung), $\equiv$CH (die Bindung zwischen dem Karbon des Ringes und R3 ist dann eine Dreifachbindung)
- R4 wird ausgewählt aus -H, -OH and =O (die Bindung zwischen dem Karbon des Rings und R4 ist dann eine Doppelbindung)

zur Verwendung bei der Behandlung von Torsades de Pointes.

2. Die Verbindung der allgemeinen Formel (I) zur Verwendung derselben zur Verhinderung des Auftretens von Episoden von torsades de pointes.

3. Die Verbindung der allgemeinen Formel (I) zur Verwendung derselben zur Verhinderung des plötzlichen Todes bei einem diabetischen Patienten, der durch das Auftreten von Torsades de Pointes verursacht wird.

4. Die Verbindung der allgemeinen Formel (I) zur Verwendung wie in einem der Ansprüche 1 bis 3 beansprucht, **dadurch gekennzeichnet, dass** der Patient eine Prädisposition für das Auftreten von Torsades de Pointes hat.

5. Die Verbindung der allgemeinen Formel (I) zur Verwendung wie in Anspruch 4 beansprucht, **dadurch gekennzeichnet, dass** der Patient einer Behandlung mit einem QT-erhöhenden Medikament folgt.

6. Die Verbindung der allgemeinen Formel (I) zur Verwendung wie in einem der Ansprüche 1 bis 5 beansprucht, **dadurch gekennzeichnet, dass** sie dem Patienten verabreicht wird, nachdem dieser eine Episode von Torsade de Pointes hatte.

7. Die Verbindung der allgemeinen Formel (I) zur Verwendung wie in Anspruch 1 beansprucht, **dadurch gekennzeichnet, daß** sie dem Patienten während eines Zeitraums von weniger als oder gleich 15 Tagen verabreicht wird, nachdem dieser eine Episode von Torsade-de-Points hatte.

8. Die Verbindung der allgemeinen Formel (I) zur Verwendung wie in einem der Ansprüche 1 und 4 bis 6 beansprucht, wobei diese von Anspruch 1 abhängig sind, **dadurch gekennzeichnet, daß** die dem Patienten verabreichte Tagesmenge der Verbindung der Formel (I) zwischen 30 $\mu$g und 3 mg liegt.

9. Die Verbindung der allgemeinen Formel (I) zu deren Verwendung nach Anspruch 2 bis 5, **dadurch gekennzeichnet, dass** die dem Patienten verabreichte Tagesmenge der Verbindung der Formel (I) zwischen 30 $\mu$g und 300 $\mu$g beträgt.

10. Die Verbindung der allgemeinen Formel (I) zur Verwendung wie in einem der Ansprüche 1 bis 7 beansprucht, **dadurch gekennzeichnet, daß** sie in einer zur oralen Verabreichung geeigneten Form vorliegt.

11. Die Verbindung der allgemeinen Formel (I) zur Verwendung wie in einem der Ansprüche 1 und 6 bis 10 beansprucht, **dadurch gekennzeichnet, daß** sie in einer zur injizierbaren Verabreichung geeigneten Form vorliegt.

12. Die Verbindung der allgemeinen Formel (I) zur Verwendung wie in einem der Ansprüche 1 bis 11 beansprucht, **dadurch gekennzeichnet, dass** die Patientin weiblich ist.

**13.** Die Verbindung zur Verwendung wie in einem der Ansprüche 1 bis 12 beansprucht, **dadurch gekennzeichnet, dass** sie aus der Gruppe ausgewählt ist, die aus Levonorgestrel, Norethisteron, Desogestrel, Etonogestrel und 19-Nortestosteron besteht.

**14.** Zusammensetzung, enthaltend eine Verbindung der allgemeinen Formel (I) und ein Androgen zu deren gleichzeitiger, getrennter oder über die Zeit verteilter Verwendung zur Behandlung oder Verhinderung von Torsades de Pointes.

**15.** Zusammensetzung zur Verwendung nach Anspruch 14, **dadurch gekennzeichnet, dass** das Androgen aus der Gruppe ausgewählt ist, die aus Testosteron, Dehydroepiandrosteron, ∆4-Androstendion, Androsteron und Dihydrotestosteron besteht.

**16.** Zusammensetzung, enthaltend eine Verbindung der allgemeinen Formel (I) und IGF-I zur gleichzeitigen, getrennten oder über die Zeit verteilten Verwendung derselben, zur Verhinderung des plötzlichen Todes bei einem diabetischen Patienten, der durch das Auftreten von Torsades de Pointes verursacht wird.

**17.** Verbindung der allgemeinen Formel (I) und ein Androgen zu deren Verwendung, zur Verhinderung des plötzlichen Todes bei einem diabetischen Patienten, der durch das Auftreten von torsades de pointes verursacht wird, wobei der Patient männlich ist.

## Claims

**1.** A compound of general formula (I)

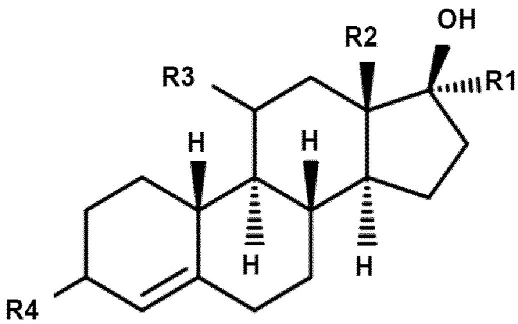

wherein

- R1 is chosen from H, $CH_3$, $CH_2$-$CH_3$, CH=$CH_2$, C≡CH
- R2 is chosen from $CH_3$, $CH_2$-$CH_3$, CH=$CH_2$, C≡CH
- -R3 is chosen from -H, -$CH_3$, =$CH_2$ (the bond between the carbon of the ring and R3 then being a double bond), ≡CH (the bond between the carbon of the ring and R3 then being a triple bond)
- -R4 is chosen from -H, -OH and =O (the bond between the carbon of the ring and R4 then being a double bond)

for use thereof for the treatment of torsades de pointes.

**2.** The compound of general formula (I) for use thereof for the prevention of the occurrence of episodes of torsades de pointes.

**3.** The compound of general formula (I) for use thereof for the prevention of sudden death in a diabetic patient caused by the occurrence of torsades de pointes.

**4.** The compound of general formula (I) for use thereof as claimed in one of claims 1 to 3, **characterized in that** the patient has a predisposition to the occurrence of torsades de pointes.

**5.** The compound of general formula (I) for use thereof as claimed in claim 4, **characterized in that** the patient is following a treatment with a QT-increasing drug.

6. The compound of general formula (I) for use thereof as claimed in one of claims 1 to 5, **characterized in that** it is administered to the patient after the latter has had an episode of torsade de pointes.

7. The compound of general formula (I) for use thereof as claimed in claim 1, **characterized in that** it is administered, for a period of less than or equal to 15 days, to the patient after the latter has had an episode of torsade de pointes.

8. The compound of general formula (I) for use thereof as claimed in one of claims 1, and 4 to 6, as regards them being dependent on claim 1, **characterized in that** the daily amount of compound of formula (I) administered to the patient is between 30 μg and 3 mg.

9. The compound of general formula (I) for use thereof as claimed in claims 2 to 5, **characterized in that** the daily amount of compound of formula (I) administered to the patient is between 30 μg and 300 μg.

10. The compound of general formula (I) for use thereof as claimed in one of claims 1 to 7, **characterized in that** it is in a form suitable for oral administration.

11. The compound of general formula (I) for use thereof as claimed in one of claims 1, and 6 to 10, **characterized in that** it is in a form suitable for injectable administration.

12. The compound of general formula (I) for use thereof as claimed in one of claims 1 to 11, **characterized in that** the patient is female.

13. The compound for use thereof as claimed in one of claims 1 to 12, **characterized in that** it is chosen from the group consisting of levonorgestrel, norethisterone, desogestrel, etonogestrel and 19-nortestosterone.

14. A composition containing a compound of general formula (I) and an androgen for use thereof simultaneously, separately or spread out over time, for the treatment or prevention of torsades de pointes.

15. The composition for use as claimed in claim 14, **characterized in that** said androgen is chosen from the group consisting of testosterone, dehydroepiandrosterone, Δ4-androstenedione, androsterone and dihydrotestosterone.

16. A composition containing a compound of general formula (I) and IGF-I for use thereof simultaneously, separately or spread out over time, for the prevention of sudden death in a diabetic patient, caused by the occurrence of torsades de pointes.

17. A compound of general formula (I) and an androgen for use thereof, for the prevention of sudden death in a diabetic patient caused by the occurrence of torsades de pointes, the patient being male.

| | Pas d'hormones | Levonorgestrel | Desogestrel | Gestodene | Drospirenone | Pvalue |
|---|---|---|---|---|---|---|
| Nombre | 207 | 137 | 41 | 51 | 62 | |
| Age (années) | 25.7 [21;41.7] $^{*,§}$ | 23 [21; 26.5] $^{§}$ | 22.8 [20.7;25.2] | 23 [21; 24.6] | 22.3 [20.7;24.7]* | <0.001 |
| Kaliémie (mmol/l) | 4.05±0,27 | 3.99±0,28 | 4±0,28 | 3.94±0,25 | 4.01±0,28 | 0.07 |
| Sotalolémie H3 (ng/ml) | 422 [340; 564] | 384 [287; 491] | 441 [282; 565] | 414[299;538] | 413 [341; 515] | 0.06 |
| QTcF de base (msec) | 393.8±16.9 | 392±14.2 | 390±12.1 | 393.7±14.3 | 394.7±15.2 | ns |
| RR de base (msec) | 902±104* | 882±114 | 884±98 | 889±96 | 857±100* | 0.04 |
| TAmp de base (µV) | 436±129 | 441±121 | 468±99 $^{\#}$ | 397±103 $^{\#}$ | 417±113 | 0.04 |
| TpTe de base (msec) | 66 [61; 72] | 66 [62; 71] | 63 [59.5; 67,5] | 67 [63; 71] | 65 [62; 68.3] | 0.06 |
| Double pic de base(%) | 0% | 0% | 0% | 0% | 0% | ns |
| ΔQTcF (msec) | 24.6±12.5* | 24.2±13.7** | 27±12.7 | 28.1±13.2 | 31.2±12.6 $^{*,**}$ | 0.003 |
| ΔQTcF (%) | 6.2±3.2* | 6.2±3.5** | 6.9±3.2 | 7.1±3.4 | 7.9±3.1 $^{*,**}$ | 0.003 |
| ΔTAmp (%) | 15.7[5.8; 27.4] | 19.6[1.7; 27.6] | 18[9.9; 25.9] | 22[13.3;31.5] | 20[10.7; 29] | 0.09 |
| ΔTpTe (%) | 12.7[6.4; 24.9] | 11.7[5.9; 21.8] | 12.3[2.6; 20] | 16.3[7; 23.1] | 15.5[9; 31] | 0.1 |
| ΔRR (%) | 19.3±11.5 | 16.5±10.7 | 16.8±9.8 | 17.8±9.5 | 18.6±12.1 | ns |
| PC1 (marqueur de l'inhibition de IKr) | 0.09[-0.58; 1.1]* | 0.22[-0.54; 1.2] | 0.27[-0.47; 1.2] | 0.52[-0.03;1.4] | 0.56[-0.13; 1.5]* | 0.03 |
| Double pic H3 (%) | 13.4% | 16.4% | 7.3 % | 23.5% | 25.8% | 0.002 |

**Figure 1**

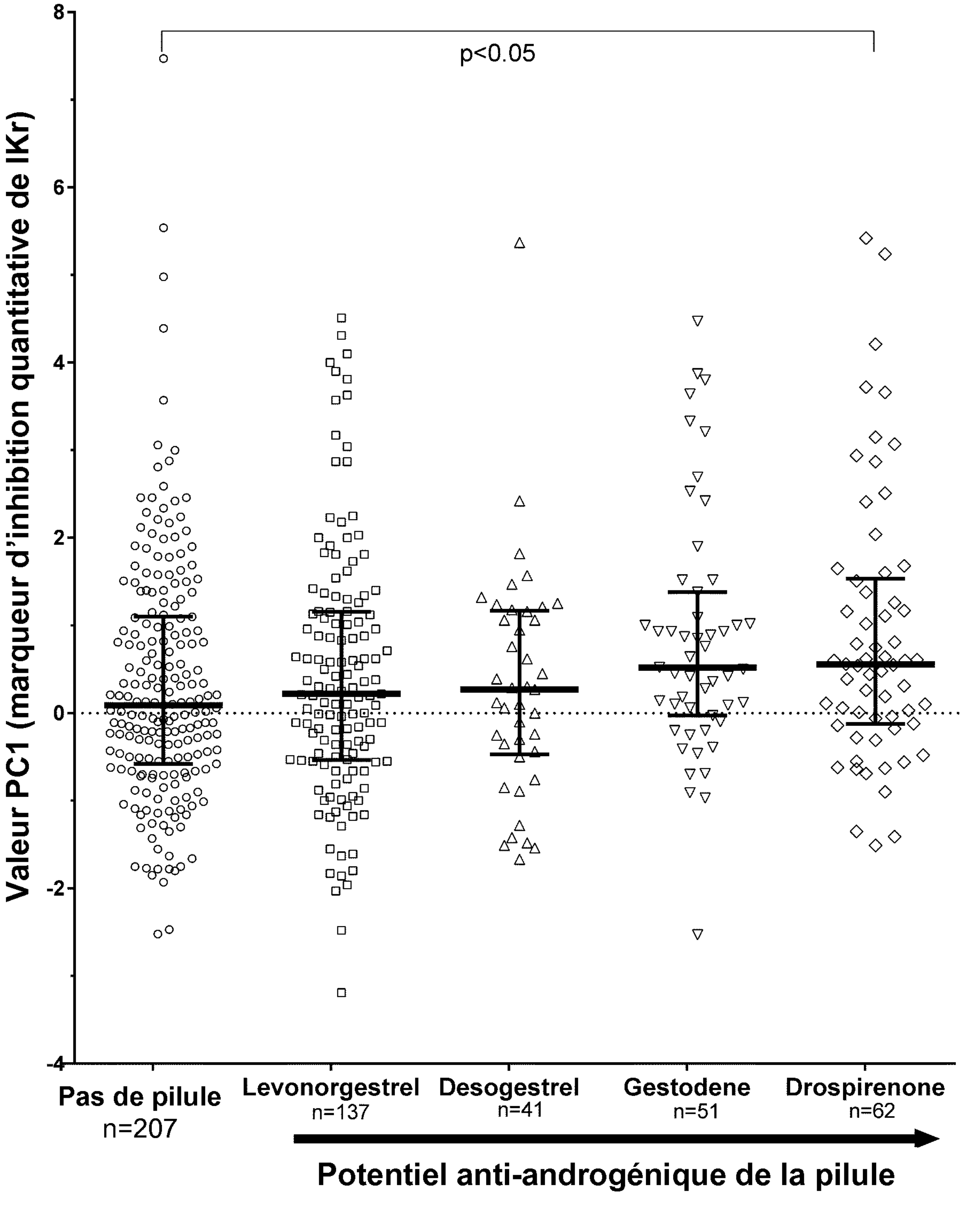

p<0.05
Valeur PC1 (marqueur d'inhibition quantitative de IKr)
8
6
4
2
0
-2
-4
Pas de pilule
n=207
Levonorgestrel
n=137
Desogestrel
n=41
Gestodene
n=51
Drospirenone
n=62
Potentiel anti-androgénique de la pilule


Figure 2

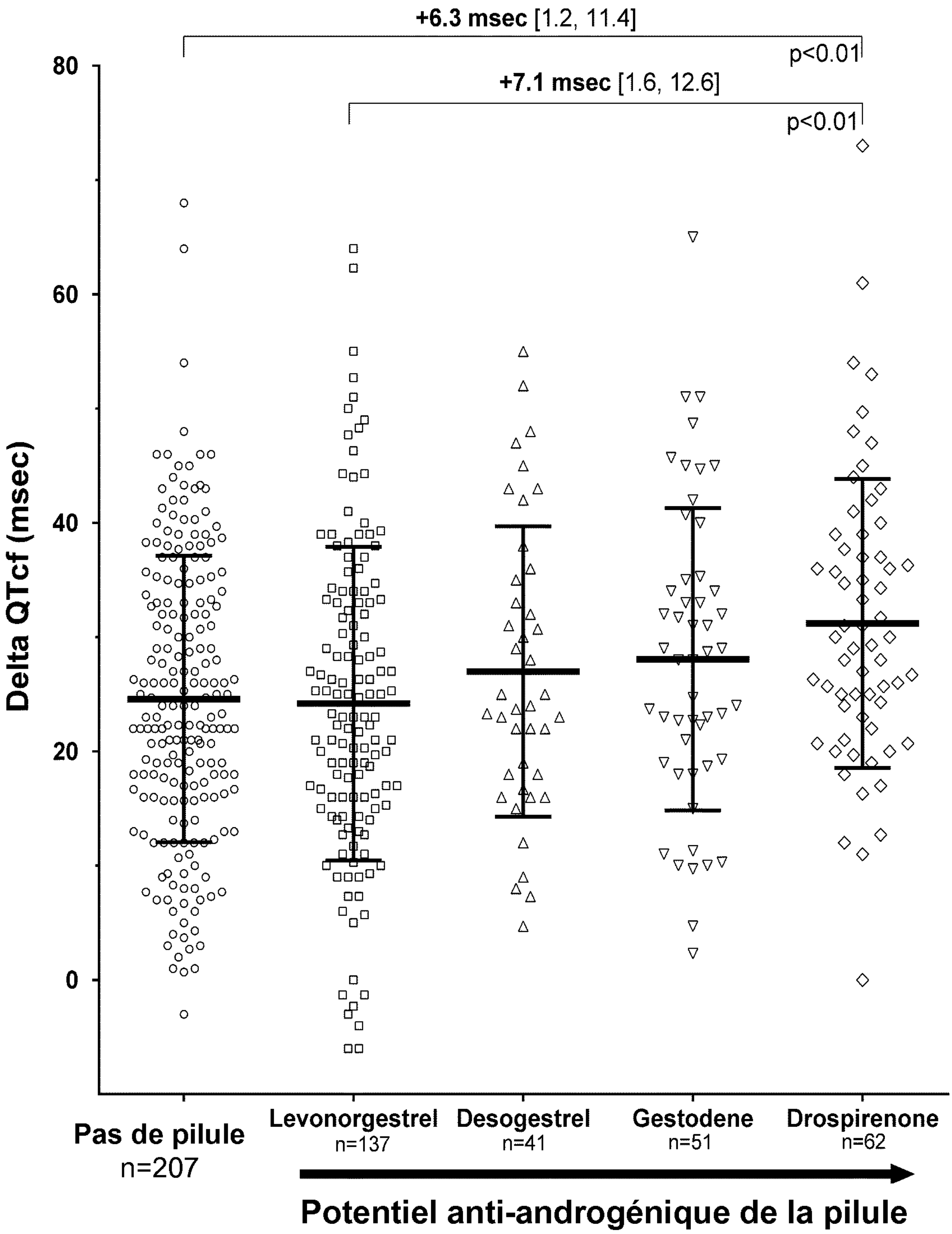

+6.3 msec [1.2, 11.4]
p<0.01
+7.1 msec [1.6, 12.6]
p<0.01
Delta QTcf (msec)
80
60
40
20
0
Pas de pilule
n=207
Levonorgestrel
n=137
Desogestrel
n=41
Gestodene
n=51
Drospirenone
n=62
Potentiel anti-androgénique de la pilule


Figure 3

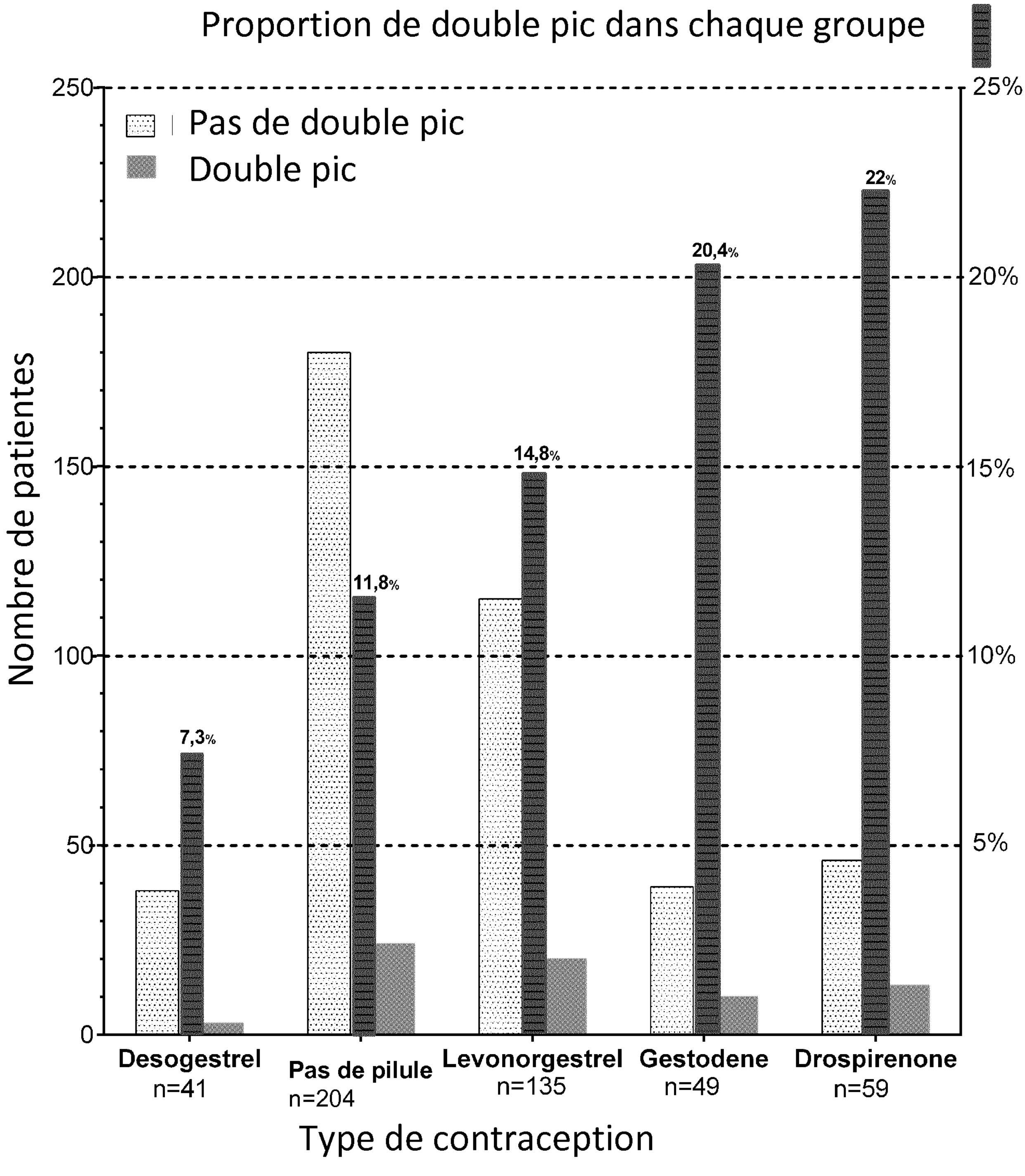
Proportion de double pic dans chaque groupe
Pas de double pic
Double pic
Nombre de patientes
250
200
150
100
50
0
25%
20%
15%
10%
5%
7,3%
11,8%
14,8%
20,4%
22%
Desogestrel
n=41
Pas de pilule
n=204
Levonorgestrel
n=135
Gestodene
n=49
Drospirenone
n=59
Type de contraception

**Figure 4**

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Littérature non-brevet citée dans la description


- **RODRIGUEZ et al.** *JAMA,* 14 Mars 2001, vol. 285 (10), 1322-6
- **ODENING et al.** *Heart Rhythm,* Mai 2012, vol. 9 (5
- **SETH et al.** *JACC,* 2007, vol. 49 (10), 1092-8
- **SEKARSKI et al.** *Paediatrica,* 2008, vol. 19 (4
- **HALIMI.** le syndrome de mort subite dans son lit, « Dead-in-bed ». *Médecine des maladies Métaboliques,* Mars 2013, vol. 7
- **SEDLAK et al.** *Ann Noninvasive Electrocardiol.,* Juillet 2013, vol. 18 (4), 389-98
- **MERIGGIOLA et al.** *J Clin Endocrinol Metab.,* Avril 2005, vol. 90 (4), 2005-14
- **ABEHSIRA et al.** *J Clin Endocrinol Metab.,* Juillet 2016, vol. 101 (7), 2776-84